# EUROPÄISCHE PATENTSCHRIFT

(11) **EP 1 476 234 B1**
(45) Veröffentlichungstag und Bekanntmachung des Hinweises auf die Patenterteilung: **03.08.2005**
(21) Anmeldenummer: 02719855.5
(22) Anmeldetag: 15.02.2002
(51) Int. Cl.: B01D 3/00

(54) **REAKTIONSKOLONNE IN SPEZIELLER KOMBINATION MIT UMLAUFVERDAMPFER**
REACTION COLUMN IN A SPECIAL COMBINATION WITH A CIRCULATION EVAPORATOR
COLONNE DE REACTION EN COMBINAISON SPECIALE AVEC UN EVAPORATEUR A CIRCULATION

(43) Veröffentlichungstag der Anmeldung: 17.11.2004
(73) Patentinhaber: BASF AKTIENGESELLSCHAFT, 67056 Ludwigshafen (DE)
(72) Erfinder: BREDEHÖFT, Jan Peter, 67459 Böhl-Iggelheim (DE); PALLASCH, Hans-Jürgen, 67063 Ludwigshafen (DE); HECK, Ludwig, E., 68535 Edingen-Neckarhausen (DE); STRÖFER, Eckhard, 68163 Mannheim (DE)
(74) Vertreter: Isenbruck, Günter, Dr.
(86) Internationale Anmeldenummer: PCT/EP2002/001658
(87) Internationale Veröffentlichungsnummer: WO 2003/068359

(56) Entgegenhaltungen:
- EP-A- 0 302 336
- DE-A- 2 410 474
- DE-A- 10 027 779
- DE-A- 19 631 332
- US-A- 4 380 615

## Beschreibung

Die vorliegende Erfindung betrifft die Verwendung einer Anordnung zur Durchführung von chemischen Reaktionen umfassend, wenigstens eine Destillationskolonne und/oder wenigstens einen Behälter sowie wenigstens einen Umlaufverdampfer, wobei diese durch entsprechende Verbindungselemente miteinander verbunden sind, zur Herstellung von Diisocyanaten.

Chemische Reaktionen bei deren Durchführung Destillationskolonnen eingesetzt werden können, sind beispielsweise aus der Polyurethan-Chemie bekannt. So kann beispielsweise eine Auftrennung des Mononitrier-Gemisches, welche bei der Herstellung von Toluylendiamin (IDA) durch die in einem ersten Schritt durchzuführende Nitrierung des Toluols zu einem Gemisch an Toluylendiamin-Isomeren anfällt, in der Regel großtechnisch nur durch mehrere Destillationsschritte erreicht werden.

Auch bei der Synthese technisch bedeutender Isocyanate, beispielsweise Toluylendiisocyanat (TDI) oder Methylendiphenyldiisocyanat (MDI) über die Phosgenierung bedient man sich verschiedenartiger Mischeinrichtungen, welche unter anderem auch Destillationseinrichtungen enthalten können.

Problematisch bei dieser Art der Synthesen ist unter anderem, daß bei ungenügender Vermischung der im einzelnen eingesetzten Reaktionskomponenten sowie ungünstigen Reaktionsbedingungen ein glatter Reaktionsablauf, das heißt optimaler Reaktionsumsatz, welcher insbesondere für die Höhe der Ausbeute entscheidend ist, häufig nicht erreicht werden kann. Die dabei sodann vermehrt anfallenden Nebenprodukte sowie unumgesetzte Edukte, welche sich auch im Reaktionssumpf ansammeln, werden bisher in einem weiteren Schritt kosten- und zeitaufwendig vom Hauptprodukt abgetrennt und aufgearbeitet (entnommen aus PUR-Kunststoffhandbuch Nr. 7, 3. Auflage, Hanser Verlag, S. 77 Kap. 3.2.2)

Eine bekannte Anordnung zur Erzielung eines verbesserten Reaktionsumsaem ist eine Vorrichtung mit einem räumlich geteilten Sumpf (heiß/kalt) innerhalb der verwendeten Reaktionskolonne. Mit Hilfe des geteilten Sumpfes ist es möglich, daß die heiße Flüssigkeit, welche aus dem Verdampfer, welcher der Reaktionskolonne zugeschaltet ist, austritt, sich nicht wieder mit dem kalten Sumpfinhalt der Reaktionskolonne vermischen kann. Im Ergebnis erhält man so zum einen einen heißen Sumpfteil, aus dem das Sumpfprodukt zur weiteren Aufarbeitung abgezogen wird, und zum anderen einen kalten Sumpfteil. In den kalten Sumpfteil wird die kalte Flüssigkeit vom untersten Boden der Reaktionskolonne, welcher sich oberhalb des Sumpfes befindet, sowie der Teil des heißen Sumpfteils, welcher aufgrund der räumlichen Anordnung von diesem überläuft, zugemischt

Als nachteilig an dieser Anordnung kann das geringe Verhältnis zwischen der volumenmäßigen Ausdehnung von heißem zu kaltem Sumpfteil angesehen werden. Die bisherigen Versuche zur Behebung dieses Nachteils scheiterten jedoch an den Grenzen, welche unter anderem durch die Bauform und Baugröße der jeweiligen Anordnung vorgegeben waren.

Das Dokument DE-A-10027779 beschreibt die Isocyanatherstellung in einer Reaktionskolonne mit Wärmeaustauscher und Zwischerhitzern.

Eine weiterhin bekannte Anordnung versucht dieses Problem durch Vergrößerung des heiβen Sumpfteils zu lösen. In dieser Anordnung wurde auf die räumliche Trennung zwischen kaltem und heißem Sumpfteil verzichtet, so daß sich der kalte Sumpfteil naturgemäß unterhalb des heißen Sumpfteils befindet Die Flüssigkeit vom untersten Boden der Reaktionskolonne wird dabei direkt vor den Stutzen, von welchem das Sumpfprodukt abgezogen werden kann und welcher sich in der Regel am Kolonnenboden, also im kalten Sumpfteil befindet, geführt

Als nachteilig an dieser Anordnung kann jedoch gesehen werden, daß die kalte Flüssigkeit beim Durchlaufen des sie führenden Rohres durch den heißen Sumpfteil bereits zu verdampfen beginnt, so daß vor dem Stutzen eine Flüssigkeit mit gasförmigen Anteilen geführt wird. Diese gasförmigen Anteile in der anschließend zu verdampfenden Flüssigkeit führten zu einer Verschlechterung des Umlaufs im angeschlossenen Verdampfer. Daher kommt es in der Regel zu Funktionsstörungen oder sogenanntem Fouling. Unter Fouling wird in diesem Zusammenhang die Bildung von unerwünschten Ablagenmgen beispielsweise an den jeweiligen Rohrinnenseiten verstanden, die bei höheren Temperaturen auf tritt.

Des weiteren beschreibt Kister (Henry Kister, Distillation operation, MacGraw-Hill, 1989, S. 97) den Einsatz eines Once-Through-Verdampfers, der zum Anfahren einer Kolonne als Naturumlaufverdampfer betrieben wird. Eine Kombination von Naturumlaufverdampfer und Once-Through-Verdampfer wird jedoch nicht erwähnt

Eine Aufgabe der vorliegenden Erfindung ist es somit, eine Anordnung bereitzustellen, mit der chemische Reaktionen, wie beispielsweise die oben beschriebene Herstellung von TDI oder MDI ohne die genannten Nachteile, bei gleichzeitiger Verbesserung des Reaktionsumsatzes, kostengünstig durchgeführt werden können.

Diese Aufgabe wird gelöst durch die Verwendung einer Anordnung zur Herstellung von Diisocyanaten umfassend wenigstens eine Destillationskolonne und/oder wenigstens einen Behälter sowie wenigstens einen Umlaufverdampfer, welche über Verbindungselemente miteinander verbunden sind, wobei Flüssigkeit vollständig oder teilweise von einem Boden oder von einem Packungsbett oder von einer Füllkörperschüttung oder von einem Flüssigkeitssammler der Destillationskolonne oder als externer Zulaufstrom unterhalb vom unteren Rohrboden des Umlaufverdampfers diesem zugeführt wird.

Besonders bevorzugt wird die Herstellung TDI und MDI durchgeführt.

Bei der verwendeten Destillationskolonne handelt es sich vorzugsweise um eine mehrstufige kontinuierlich arbeitende Bodenkolonne.

Alternativ dazu können ebenfalls Rohrkolonnen, Staubodenkolonnen, Rieselfilmkolonnen, Sprühkolonnen, Blasensäulen, Füllkörperkolonnen, Packungskolonnen und Anstaupakkungskolonnen eingesetzt werden, die kontinuierlich als Semibatch oder im Batch betrieben werden.

Prinzipiell strömt in senkrecht stehenden Kolonnen in der Mehrzahl aller Fälle aus energetischen Gründen die flüssige Phase von oben nach unten und die gasförmige Phase von unten nach oben. Für die vorliegende Erfindung werden vorzugsweise senkrecht stehende Kolonnen eingesetzt, deren Phasen im Gegenstrom, im Kreuzstrom, im Gleichstrom, im Kreuz-Gleichstrom sowie im Kreuz-Gegenstrom geführt werden können, wobei die jeweilige Strömungsform durch die Einbauten bestimmt wird.

Unter Flüssigkeit wird im Rahmen der vorliegenden Erfindung das in der Destillationskolonne gewonnene Kondensat und/oder ein flüssiger Zulauf verstanden, welches vom untersten der in der Destillationskolonne vorgesehenen Böden abgezogen wird. Ein Boden in einer Kolonne ist in der Regel dadurch charakterisiert, daß zwischen dem von einem Boden aufsteigenden Dampf und der von ihm abfließenden Flüssigkeit thermisches Gleich gewicht besteht. Es sind jedoch auch Böden denkbar, auf denen kein thermodynamisches Gleichgewicht besteht Bei den vorzugsweise verwendeten Böden sind Böden mit Flüssigkeitszwangsführung und Böden ohne Flüssigkeitszwangsführung zu unterscheiden. Des weiteren können die Böden zur Erhöhung der Flexibilität als Austauschböden gestaltet sein. Je nach vorgegebenem Strömungsweg, kommen bei Böden mit Flüssigkeitszwangsführung beispielsweise Querstromböden, Umlenkstromböden oder Radiaistmmböden zur Anwendung. Anstelle oder zusätzlich zu diesen Böden kann auch ein Packungsbett oder eine Füllkörperschüttung vorgesehen sein.

Bei der Füllkörperschüttung handelt es sich vorzugsweise um eine gleichmäßig über die gesamte Kolonnenhöhe verteilte, mehr oder weniger regelmäßig angeordnete Füllung, die auf einem Tragrost ruht Die Schüttungen bestehen dabei aus regelmäßig geformten Körpern, wobei die Formengrößen der Füllkörper sehr vielfältig und darauf gerichtet sind, eine möglichst große Oberfläche zu realisieren und einen großen freien Durchtrittsquerschnitt für das Gas offenzulassen. Als Füllkörperarten kommen vorzugsweise Ringkörper wie Raschig - Ringe und Pall - Ringe und Sattelkörper wie Intalox - Sättel und Berl - Sättel zum Einsatz, welche ihrerseits aus verschiedenen Materialien wie beispielsweise Keramik, Metall, Glas und Kunststoff bestehen können.

Packungen, auch im ganzen als Packungsbett bezeichnet, stellen im Gegensatz zu Füllkörperschüttungen mit unregelmäßiger Struktur, geordnete Kolonnenfüllungen dar. Die Auswahl des Werkstoffs der Packungen wird hauptsächlich von Korrosionseigenschaften, aber auch von strömungstechnischen Parametern bestimmt. Die Packungen können unterschiedlich, beispielsweise in Form von unperforierten Platten und Bändern ohne und mit Oberflächenstruktur (z.B. Siebplatten, Streckmetall) sowie aus Geweben, Gewirken, Gestricken und Geflechten gestaltet sein.

Die erfindungsgemäße Anordnung ist weiterhin dadurch gekennzeichnet, daß der Umlauf verdampfer ein Naturumlaufverdampfer ist.

Die im Rahmen der Erfindung zur Anwendung kommenden Verdampfer können in der Regel alle bekannten Verdampfer sein. Bevorzugt werden jedoch Zwangsumlaufverdampfer wie beispielsweise Kletterfilin-, Zentrifugal- und Rotationsverdampfer sowie Umlaufverdampfer wie beispielsweise Robert-Verdampfer, Schrägrohr- und Langrohrverdampfer sowie Natummlaufverdampfer verwendet.

Bei dem besonders bevorzugt vorgesehenen Once-Through-Verdampfer handelt es sich um eine Vorrichtung zur Verdampfung von Flüssigkeiten, wobei die Flüssigkeit beim einmaligen Durchgang durch den Apparat ganz oder teilweise verdampft wird.

Bei dem bezeichneten Natiuvmlaufverdampfer handelt es sich um einen Verdampfer, welcher ohne die Verwendung von Pumpen funktioniert, wobei das zu verdampfende Fluid in den Rohren eines Rohrbündels meist durch kondensierenden Wasserdampf aufgeheizt und teilweise verdampft wird. Durch die Dichtedifferenz des Fluids zwischen Zulauf und Rohrbündel entsteht ein natürlicher Umlauf.

Die Verbindungselemente zwischen Destillationskolonne und Verdampfer werden bevorzugt in Form von Rohrleitungen gestaltet. Alternativ können auch Leitbleche, Schächte oder Schläuche eingesetzt werden.

In einer weiteren bevorzugten Ausfiihmngsform wird die Flüssigkeit vom Zulauf der Destillationskolonne oder des Behälters abgezogen und unterhalb des unteren Rohrbodens des Umlaufverdampfers diesem zugeführt.

Erfindungsgemäß ist die Anordnung in einer bevorzugten Ausführungsform weiterhin dadurch gekennzeichnet, daß die dem Umlaufverdampfer zugeführte Flüssigkeit vorzugsweise über wenigstens ein Zulauf- und/oder Verteilersystem unterhalb des unteren Rohrbodens des Umlaufverdampfers mit einer vom heißen Sumpfteil der Destillationskolonne oder des Behälters zirkulierenden Flüssigkeit gemischt wird.

Als Zulauf- und/oder Verteilersystem kommen bevorzugt Einsteckrohre, direkte Einspeisungen, tangentiale Einspeisungen, Düsen- oder Strahleinheiten zur Anwendung.

Besonders bevorzugt handelt es sich dabei um eine Ringleitung unterhalb des unteren Rohrbodens des Umlaufverdampfers oder den Einsatz von statischen Mischelementen bei der direkten Einspeisung.

Bei einer weiteren bevorzugten Ausfiihrungsform der vorliegenden Erfindung weist die Destillationskolonne oder der Behälter einen geteilten Sumpf auf, wobei der geteilte Sumpf wenigstens einen kalten und wenigstens einen heißen Sumpfteil aufweist.

Diese Teilung kann dabei durch Trennelemente wie z.B. eine Trennwand erfolgen, wenn ein räumlich zusammenhängender Sumpflsereich geteilt werden soll oder aber auch durch räumliche Trennung, so daß die Sumpfteile voneinander derart beabstandet sind, daß eine gegenseitige Temperaturbeeinflussung möglichst verhindert wird. Die Aufteilung des Sumpfes kann grundsätzlich auch in mehr als zwei Teile erfolgen, wobei die Größe der einzelnen Sumpfteile frei bestimmbar ist.

Eine weitere Ausgestaltung der vorliegenden Erfindung ist dadurch gekennzeichnet, daß bei Anwendung der erfindungsgemäßen Anordnung auf Gemische, in denen Reaktionen ablaufen, bei gleicher Baugröße eine Maximierung des heißen und eine Minimierung des kalten Sumpfteils erzielt wird.

Gemische sind im Rahmen der vorliegenden Erfindung sowohl homogene wie heterogene Mischungen einer oder mehrerer reiner Komponenten. Bei den homogenen Gemischen handelt es sich vorzugsweise um Flüssigkeiten. Bei den heterogenen Gemischen handelt es sich bevorzugt um eine oder mehrere flüssige Phasen, eine gasförmige Phase und/oder eine oder mehrere feste Phasen.

Unter der Baugröße ist die Baugröße der Anordnung zur Durchführung eines vergleichbaren Reaktionsumsatzes zu verstehen. Eine geringere Baugröße würde daher weniger Inhalt an toxischen oder anderweitig gefährlichen Substanzen bedeuten.

Die erfindungsgemäße Anordnung bietet in der Form der Zusammenschaltung den Vorteil, daß die kalte Flüssigkeit, die beim Mischen mit der heißen Flüssigkeit verdampft, den Naturumlauf stabilisiert und den Wärmeübergang verbessert, womit einem Fouling entgegengewirkt wird.

Neben der Vermeidung einer Zweiphasenströmung im Sumpf der Kolonne erreicht man bei dieser Kombination durch die Mischung der beiden Flüssigkeiten eine geringere Ein und Austrittstemperatur und damit, bei vorgegebener Heizmitteltemperatur, eine größere Temperaturdifferenz am Verdampfer.

Die Austrittstemperatur aus dem bevorzugt zur Anwendung kommenden Natunjimlaufverdampfer entspricht dabei vorzugsweise der Sumpftemperatur und ist damit identisch der Verdampferaustrittstemperatur im Once-Trough-Verdampfer. Die Eintrittstemperatur des Naturumlaufverdampfers liegt bevorzugt zwischen Sumpftemperatur und Ablauftemperatur und wird bestimmt durch die sich einstellende Umlaufinenge.

Die Kombination von Naturumlauf- und Once-Through-Verdampfer insbesondere für das TDI-Verfahren führt zu einer Verbesserung des Reaktionsumsatzes durch Maximierung des heißen Sumpfteils. Dadurch kann entweder die Verweilzeit für das Phosgen gesenkt oder das Foulingrisiko reduziert werden oder der Umsatz zum Wunschprodukt TDI erhöht werden, was einer größeren Ausbeute entspricht

Besonders vorteilhaft ist die Anwendung der vorliegenden Erfindung bei kinetisch kontrollierten Stoffumsetzungen, endothermen Gleichgewichtsreaktionen oder Stofftransporten, da die höheren Temperaturen in diesen Fällen bei gleichem Flüssigkeitsvolumen zu schnelleren und besseren Reaktionsumsätzen führen.

Weiterhin kann gemäß der vorliegenden Erfindung das Verfahren zur Herstellung von Toluylendiisocyanat (TDI) aus Toluylendiamin (TDA) und seinem Amin Hydrochlorid und Phosgen mit Hilfe der erfindungsgemäßen Anordnung durchgeführt werden.

Ebenso ist mit Hilfe dieser Anordnung das Verfahren zur Herstellung von Methylendiphenyldiisocyanat (MDI) aus Methylendiphenyldiamin (MDA) und seinem Amin Hydrochlorid und Phosgen durchführbar.

Erfindungsgemäß ist ebenso die Verwendung der Anordnung zur Durchführung des Verfahrens zur Herstellung von TDI oder MDI vorgesehen.

Die vorliegende Erfindung wird im folgenden an einer bevorzugten Ausführungsform anhand der beigefügten Zeichnung näher erläutert Dabei zeigt

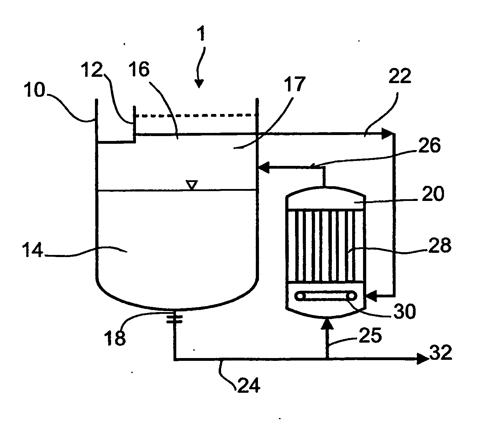

Fig. 1 eine schematische Darstellung einer erfindungsgemäßen Anordnung.

Die in Figur 1 dargestellte Anordnung 1 zeigt eine bevorzugte Ausführungsform der vorliegenden Erfindung. Dabei umfaßt die Anordnung 1 zur Durchführung des TDI-Verfahrens eine Destillationskolonne 10 mit einem untersten Boden 12 und einem heißen Sumpfteil 14 sowie einem Ablauf 16 für kalte Flüssigkeit, einen Bradenraum 17 und einem Ablauf 18 für heiße Flüssigkeit, einen Naturumlaufverdampfer 20 und entsprechende Verbindungsrohrleitungen 22 und 24 sowie ein Zulauf 25 und ein Auslaufrohr 26 des Naturumlaufverdampfers 20 zur Verbindung von Destillationskolonne 10 und Naturumlaufverdampfer 20.

Der Naturumlaufverdampfer 20 umfaßt seinerseits ein Verdampferrohrbündel 28, einen Verteilerring 30 sowie den Zulauf 25 und das Auslaufrohr 26.

Während des TDI-Verfahrens wird Flüssigkeit aus dem heißen Sumpfteil 14 der Destillationskolonne 10 durch den Ablauf 18, die Verbindungsrohrleitung 24 und den Zulauf 25 dem Verteilerring 30 zugeführt. Gleichzeitig wird die auf dem untersten Boden 12 der Destillationskolonne 10 aufgefangene Flüssigkeit über den Ablauf 16 aus der Destillationskolonne 10 abgezogen und über die Verbindungsrohrleitung 22 ebenfalls dem Verteilerring 30 des Naturumlaufverdampfers 20 zugeführt.

Diese beiden dem Verteilerring 30 des Naturumlaufverdampfers 20 zugeführten Flüssigkeiten werden vorzugsweise am Verteilerring 30 gemischt. Dabei verdampft die kalte Flüssigkeit und stabilisiert damit den Naturumlauf und verbessert gleichzeitig den Wärmeübergang.

Der eigentliche Verdampfungsprozeß im Naturumlaufverdampfer 20 erfolgt durch Heizung des Verdampferrohrbündels 28, wobei die in die einzelnen Rohre des Verdampferrohrbündels 28 vom Verteilerring 30 eingespeiste Flüssigkeit erhitzt wird. Ist die Wandtemperatur der Rohre hoch genug, so beginnt die Flüssigkeit in den Rohren des Verdampferrohrbündels 28 zu sieden, indem zuerst Blasen an der Rohrwand entstehen. Sie lösen sich ab und streben, bedingt durch den Auftrieb, dem oberen Rohrende zu, um über das Auslaufrohr 26 als Dampf in den Brüdenraum 17 einzutreten. Durch die Bildung von Dampfblasen verringert sich die mittlere Dichte in den einzelnen Rohren des Verdampferrohrbündels 28, so daß der hydrostatische Druck in den Rohren kleiner als der im heißen Sumpfteil 14 der Destillationskolonne 10 wird. Diese Differenz im hydrostatischen Druck bewirkt, daß sich ein Flüssigkeitsstrom vom heißen Sumpfteil 14 der Destillationskolonne 10 zum Verdampferrohrbündel 28 des Naturumlaufverdampfers 20 bewegt, um die Druckdifferenz auszugleichen. Der Flüssigkeitsstrom vom heißen Sumpfteil 14 der Destillanonskolonne 10 zum Verdampferrohrbündel- 28 des Naturumlaufverdampfers 20 hebt das Zweiphasengemisch in den einzelnen Verdampferrohren über den oberen Rohrboden hinaus bis in den Brüdenraum 17 an, wobei die gebildeten Dampfblasen Flüssigkeitsteile mit sich reißen. Diese Tröpfchen werden durch die Schwerkraft oder einen geeigneten Abscheider vom Brüdenraum 17 getrennt und fallen in den heißen Sumpfteil 14 der Destillationskolonne 10 zurück. Somit entsteht ein selbsttätiger Umlaufstrom.

Der Ablauf 32 dient als Sumpfabzug. Durch ihn kann mengengeregelt das Sumpfprodukt abgezogen werden, um beispielsweise in einem Nachreaktor weiterbehandelt oder aufgearbeitet zu werden.

## Patentansprüche

1. Verwendung einer Anordnung (1) zur Herstellung von Diisocyanaten umfassend wenigstens eine Destillationskolonne (10) und/oder wenigstens einen Behälter sowie wenigstens einen Umlaufverdampfer (20), welche über Verbindungselemente miteinander verbunden sind, wobei Flüssigkeit vollständig oder teilweise von einem Boden (12) oder von einem Packungsbett oder von einer Füllkörperschüttung oder von einem Flüssigkeitssammler der Destillationskolonne (10) oder als externer Zulaufstrom unterhalb vom unteren Rohrboden des Umlaufverdampfers (20) diesem zugeführt wird.

2. Verwendung (1) nach Anspruch 1, **dadurch gekennzeichnet, daß** der Umlaufverdampfer (20) ein Naturumlaufverdampfer ist.

3. Verwendung (1) nach Anspruch 1 oder 2, **dadurch gekennzeichnet, daß** die Flüssigkeit vom Zulauf der Destillationskolonne (10) oder des Behälters abgezogen und unterhalb des unteren Rohrbodens des Umlaufverdampfers (20) diesem zugeführt wird.

4. Verwendung (1) nach einem der vorhergehenden Ansprüche, **dadurch gekennzeichnet, daß** die Destillationskolonne (10) oder der Behälter einen geteilten Sumpf aufweist.

5. Verwendung (1) nach einem der vorhergehenden Ansprüche, **dadurch gekennzeichnet, daß** der geteilte Sumpf wenigstens einen kalten und wenigstens einen heiβen Sumpfteil aufweist.

6. Verwendung (1) nach Anspruch 5, **dadurch gekennzeichnet, daß** bei gleicher Baugröße eine Maximierung des heißen und eine Minimierung des kalten Sumpfteils erzielt wird.

7. Verwendung (1) nach einem der vorhergehenden Ansprüche, **dadurch gekennzeichnet, daß** die dem Umlaufverdampfer (20) zugeführte Flüssigkeit vorzugsweise über wenigstens ein Verteilersystem (30) unterhalb des unteren Rohrbodens desselben mit einer vom heißen Sumpfteil (14) der Destillationskolonne (10) oder des Behälters zirkulierenden Flüssigkeit gemischt wird.

8. Verwendung nach einem der vorhergehenden Ansprüche zur Herstellung von Toluylendiisocyanat (TDI) aus Toluylendiamin (TDA) und Phosgen.

9. Verwendung nach einem der Ansprüche 1 bis 7 zur Herstellung von Methylendiphenyldiisocyanat (MDI) aus Methylendiphenyldiamin (MDA) und Phosgen.

## Claims

1. The use of an arrangement (1) for the preparation of diisocyanates, comprising at least one distillation column (10) and/or at least one container and at least one circulation evaporator (20), which are connected to one another via connecting elements, liquid being completely or partly fed from a tray (12) or from a stacked packing bed or from a dumped packing bed or from a liquid collector of the distillation column (10) or as an external feed stream below the lower tube sheet of the circulation evaporator (20) to said evaporator.

2. The use (1) as claimed in claim 1, wherein the circulation evaporator (20) is a natural circulation evaporator.

3. The use (1) as claimed in claim 1 or 2, wherein the liquid is taken off from the feed of the distillation column (10) or of the container and is fed to the circulation evaporator (20) below the lower tube sheet of said evaporator.

4. The use (1) as claimed in any of the preceding claims, wherein the distillation column (10) or the container has a divided bottom.

5. The use (1) as claimed in any of the preceding claims, wherein the divided bottom has at least one cold and at least one hot bottom part.

6. The use (1) as claimed in claim 5, wherein maximization of the hot and minimization of the cold bottom part are achieved with the same construction size.

7. The use (1) as claimed in any of the preceding claims, wherein the liquid fed to the circulation evaporator (20) is mixed, preferably via at least one distributor system (30) below the lower tube sheet of said evaporator, with a liquid circulating from the hot bottom part (14) of the distillation column (10) or of the container.

8. The use as claimed in any of the preceding claims for the preparation of tolylene diisocyanate (TDI) from tolylenediamine (TDA) and phosgene.

9. The use as claimed in any of claims 1 to 7 for the preparation of methylenediphenyl diisocyanate (MDI) from methylenediphenyldiamine (MDA) and phosgene.

## Revendications

1. Utilisation d'un dispositif (1) pour la préparation de diisocyanates, comprenant au moins une colonne de distillation (10) et/ou au moins un récipient ainsi qu'au moins un évaporateur à circulation (20) qui sont reliés mutuellement par des éléments de liaison, du liquide étant totalement ou partiellement amené à l'évaporateur à circulation (20) en dessous du plateau à tubes inférieur de celui-ci depuis un plateau (12) ou un lit de garnissage ou un chargement de corps de remplissage ou un collecteur de liquide de la colonne de distillation (10) ou sous la forme d'un courant d'alimentation externe.

2. Utilisation (1) suivant la revendication 1, **caractérisée en ce que** l'évaporateur à circulation (20) est un évaporateur à circulation naturelle.

3. Utilisation (1) suivant la revendication 1 ou 2, **caractérisée en ce que** le liquide est soutiré de l'alimentation de la colonne de distillation (10) ou du récipient et est amené à l'évaporateur à circulation (20) en dessous du plateau à tubes inférieur de celui-ci.

4. Utilisation (1) suivant l'une des revendications précédentes, **caractérisée en ce que** la colonne de distillation (10) ou le récipient présente un fond divisé.

5. Utilisation (1) suivant l'une des revendications précédentes, **caractérisée en ce que** le fond divisé présente au moins une partie de fond froide et au moins une partie de fond chaude.

6. Utilisation (1) suivant la revendication 5, **caractérisée en ce que**, pour une même grandeur de construction, on obtient une maximisation de la partie de fond chaude et une minimisation de la partie de Fond froide.

7. Utilisation (1) suivant l'une des revendications précédentes, **caractérisée en ce que** le liquide amené à l'évaporateur à circulation (20) est mélangé à un liquide circulant depuis la partie de Fond chaude (14) de la colonne de distillation (10) ou du récipient, de préférence par l'intermédiaire d'au moins un système distributeur (30) situé en dessous du plateau à tubes inférieur de celui-ci.

8. Utilisation suivant l'une des revendications précédentes pour la préparation de diisocyanate de toluylène (TDI) à partir de toluylènediamine (TDA) et de phosgène.

9. Utilisation suivant l'une des revendications 1 à 7, pour la préparation de diisocyanate de méthylènediphényle (MDI) à partir de méthylènediphényldiamine (MDA) et de phosgène.
